(19) Europäisches Patentamt
European Patent Office
Office européen des brevets

(11) Veröffentlichungsnummer: **0 151 927**
**A1**

# (12) EUROPÄISCHE PATENTANMELDUNG

(21) Anmeldenummer: 85100117.2

(22) Anmeldetag: 07.01.85

(51) Int. Cl.⁴: C 07 D 251/70

(30) Priorität: 19.01.84 DE 3401675

(43) Veröffentlichungstag der Anmeldung: 21.08.85 Patentblatt 85/34

(84) Benannte Vertragsstaaten: BE DE FR GB IT

(71) Anmelder: BAYER AG
Konzernverwaltung RP Patentabteilung
D-5090 Leverkusen 1 Bayerwerk(DE)

(72) Erfinder: Hajek, Manfred, Dr.
Hahnenweg 1
D-5000 Köln 80(DE)

(72) Erfinder: Salzburg, Herbert, Dr.
Hahnenweg 3
D-5000 Köln 80(DE)

(72) Erfinder: Ziemann, Heinz, Dr.
Am Wiesenberg 10
D-5653 Leichlingen 1(DE)

## (54) Neue Polyamine und ein Verfahren zu ihrer Herstellung.

(57) Neue, N,N',N"-Tris-(aminoalkyl)-melamine und ein Verfahren zu ihrer Herstellung durch katalytische Hydrierung der ihnen zugrunde liegenden Trinitrile. Die neuen Polyamine eignen sich u.a. als Vernetzer für Epoxidharze oder für Isocyanatgruppen aufweisende Verbindungen.

EP 0 151 927 A1

Croydon Printing Company Ltd.

0151927

BAYER AKTIENGESELLSCHAFT 5090 Leverkusen, Bayerwerk

Konzernverwaltung RP
Patentabteilung Wr/m-c

# Neue Polyamine und ein Verfahren zu ihrer Herstellung

Gegenstand der Erfindung sind neue Polyamine der Formel

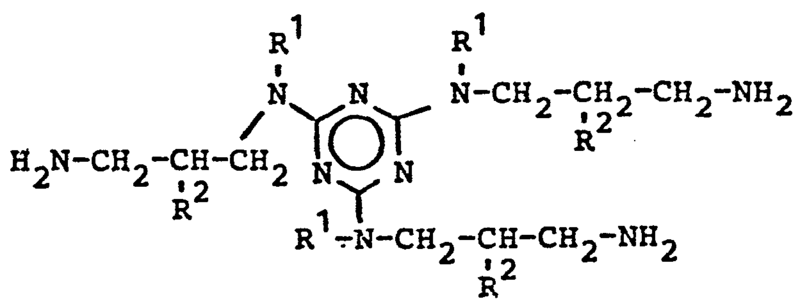

in welcher

$R^1$ für einen Alkyl-, Cycloalkyl- oder Arylrest steht und

$R^2$ für Wasserstoff oder einen Alkylrest steht.

Gegenstand der Erfindung ist auch ein Verfahren zur Herstellung der erfindungsgemäßen Polyamine, welches dadurch gekennzeichnet ist, daß man Trinitrile der Formel

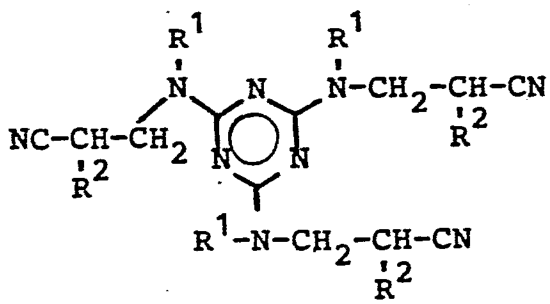

in welcher

$R^1$ und $R^2$ die vorstehend genannte Bedeutung haben,

in an sich bekannter Weise katalytisch unter Überführung der Nitrilgruppen in Aminogruppen hydriert.

Die neuen, erfindungsgemäßen Triamine stellen u.a. wertvolle Vernetzer für Epoxidharze oder für Isocyanatgruppen aufweisende Verbindungen dar. Außerdem eignen sich die neuen Polyamine als Ausgangsmaterialien bei der Herstellung von Polyamidkunststoffen.

Die einwandfreie Herstellbarkeit der erfindungsgemäßen Verbindungen durch das erfindungsgemäße Verfahren ist überraschend, da einerseits aus Ing. Eng. Chem. Prod. Res. Develop. 6, 142 (1967) bekannt war, daß bei der Hydrierung von Trinitrilen mit der Bildung von Oligomeren durch Kondensation gerechnet werden muß, so daß die Herstellung einheitlicher Verbindungen in der Regel nicht möglich ist, und da andererseits ebenfalls bekannt war, daß Triazine starke Katalysatorengifte darstellen

und die Hydrierung von Triazinderviaten demzufolge problematisch ist (vgl. z.B. M. Freifelder: "Practical Catalytic Hydrogenation" S. 622; Wiley Interscience, Wiley & Sons).

Die bei dem erfindungsgemäßen Verfahren einzusetzenden Trinitrile sind beispielsweise durch die an sich bekannte Kondensationsreaktion von Cyanurchlorid mit Anlagerungsprodukten von primären Aminen an Acrylnitril bzw. 2-substituierten Acrylnitrilen wie z.B. Methacrylnitril oder durch Anlagerung derartiger olefinisch ungesättigter Nitrile an N,N',N"-trisubstituierte Melamine zugänglich.

Die folgende Übersicht verdeutlicht die Synthesen:

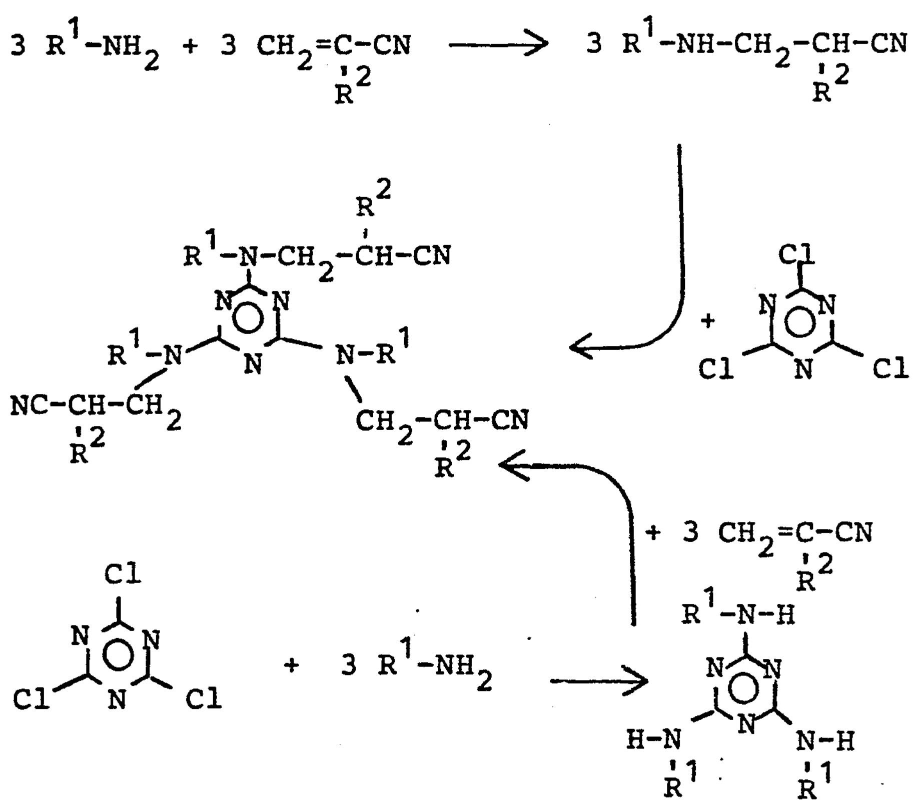

So kann beispielsweise die Anlagerung des primären Amins an das, gegebenenfalls 2-substituierte Acrylnitril unter Verwendung stöchiometrischer Mengen der Reaktionspartner bei 20 bis 120°C in Gegenwart geeigneter Lösungsmittel wie z.B. Toluol, Dioxan, Acetonitril oder in Substanz gemäß der in Org. Reactions 5, 79 - 135 (1949) beschriebenen Verfahrensweise erfolgen. Die Umsetzung des Anlagerungsprodukts mit Cyanurchlorid kann dann beispielsweise unter Verwendung stöchiometrischer Mengen der Reaktionspartner bei 0°C bis 120°C in geeigneten Lösungsmitteln wie z.B. Wasser, Dioxan, Aceton, Toluol in Analogie zu der in J. Am. Chem. Soc. 73, 2984 (1951) beschriebenen Arbeitsweise vorgenommen werden.

Bevorzugte Ausgangsmaterialien zur Herstellung der Trinitrile nach der letztgenannten Methode sind beispielsweise folgende Verbindungen:

a) beliebige primäre Alkyl-, Cycloalkyl- oder Arylamine, insbesondere Alkylamine mit 1 bis 6 Kohlenstoffatomen, wie z.B. Methylamin, n-Propylamin, Isopropylamin, n-Butylamin, Isobutylamin, n-Hexylamin, Cyclohexylamin oder Anilin. Besonders bevorzugt werden Alkylamine der genannten Art mit 1 bis 4 Kohlenstoffatomen eingesetzt.

b) Gegebenenfalls in 2-Stellung alkylsubstituierte Acrylnitrile wie insbesondere Acrylnitril oder Methacrylnitril.

c) Cyanurchlorid.

Zur Durchführung des erfindungsgemäßen Verfahrens werden die Trinitrile in einem polaren Lösungsmittel mit einer Dielektrizitätskonstante von $>7$ (DIN 53 483) wie z.B. Methanol, Ethanol, Wasser, wäßrige Essigsäure und anderer Gemische aus z.B. den genannten nach an sich bekannter Methode mit Wasserstoff in Gegenwart von Ammoniak in einem Autoklaven durchgeführt. Die Temperatur soll dabei in einem Bereich zwischen 60°C und 180°C, bevorzugt zwischen 85°C und 160°C, liegen. Besonders glatt verläuft die Reaktion zwischen 100°C und 150°C.

Während der Hydrierung wird im Autoklaven vorzugsweise ein Druck von 100 - 200 bar, insbesondere von 145 - 180 bar aufrechterhalten.

Die Hydrierung erfolgt vorzugsweise in Gegenwart eines Katalysators. Raney-Nickel und Raney-Kobalt eignen sich besonders gut, auch Nickel-Eisen oder Kobalt-Eisen-Katalysatoren sowie Katalysatoren aus der Platin-Gruppe sind geeignet.

Den gemachten Ausführungen bezüglich der geeigneten Ausgangsmaterialien entsprechend, handelt es sich bei den bevorzugten erfindungsgemäßen Triaminen um solche der

obengenannten allgemeinen Formel, für welche

$R^1$ für einen Alkylrest mit 1 bis 6 Kohlenstoffatomen, insbesondere 1 bis 4 Kohlenstoffatomen, einen Cyclohexylrest oder einen Phenylrest steht, und

$R^2$ für Wasserstoff oder eine Methylgruppe steht.

Die erfindungsgemäßen Triamine fallen in flüssiger Form und in guten Ausbeuten an und werden durch Abfiltrieren des Katalysators und Verdampfen des Lösungsmittels gewonnen. Destillation im Hochvakuum liefert besonders reine Produkte, ebenfalls die thermische Zersetzung des Kohlensäure-Salzes.

Beispiel 1

a) Herstellung des Ausgangsmaterials

N,N',N"-Tris-(2-cyanoethyl)-N,N',N"-trimethyl-melamin:

240 g (10 Mol) frisch destilliertes 3-(Methylamino)-propionsäurenitril werden in 2 l Ethanol/Wasser (Volumenverhältnis = 1:1) mit 500 g (5 Mol) Natriumcarbonat unter Rühren auf -5°C bis -10°C gebracht und bei dieser Temperatur eine Lösung von 555 g (3 Mol) Cyanurchlorid in 2 l Aceton zugetropft. Nach anfänglicher exothermer Reaktion wird schneller zugetropft und dann bei ca. 0°C bis 5°C 3 Stunden und unter langsamer Erwärmung auf 60°C 8 - 10 Stunden nachgerührt. Nach Ende der Reaktion wird auf ca. 5°C bis 10°C abgekühlt und der Niederschlag abfiltriert.

Nach dreimaligem Waschen mit je 200 ml Wasser und anschließendem Trocknen erhält man 905 g (92,3 %) Produkt; Schmp. 173°C.

b) N,N',N"-Tris-(3-aminopropyl)-N,N',N"-trimethyl-melamin:

100 g Tris-Nitril (0,31 Mol) aus Beispiel 1 a) werden in 500 ml Wasser und 500 ml Methanol mit 600 ml flüssigem Ammoniak und 40 g Raney-Nickel auf 130°C gebracht und 145 bar Wasserstoff aufgedrückt. Nach 3,5 Stunden wird nach Abkühlen und Entspannen das Gemisch durch Filtrieren vom Katalysator befreit; dieser kann zur Reaktion wiederverwandt werden. Falls nötig, wird die Lösung des Produktes mit Aktivkohle entfärbt und anschließend bei 100°C/25 mm Hg eingeengt.

Man erhält 94,2 g farblosen Sirup (89,8 %). Besonders reines Tris-Amin erhält man, wenn der ausgefallene Rohsirup bei 0,1 mm/250°C destilliert wird. Das Kohlensäure-Salz hat einen Schmp. von 92°C ($CO_2$-Abspaltung).

Beispiel 2

100 g N,N',N"-Tris-(2-methyl-2-cyano-ethyl)-N,N',N"-trimethyl-melamin (0,27 Mol) werden wie in Beispiel 1 b) angegeben hydriert. Man erhält 90 g (87,8 %) N,N',N"-Tris-(2-methyl-3-amino-propyl)-N,N',N"-trimethyl-melamin als farblosen Sirup.

Patentansprüche:

1. Polyamine der Formel

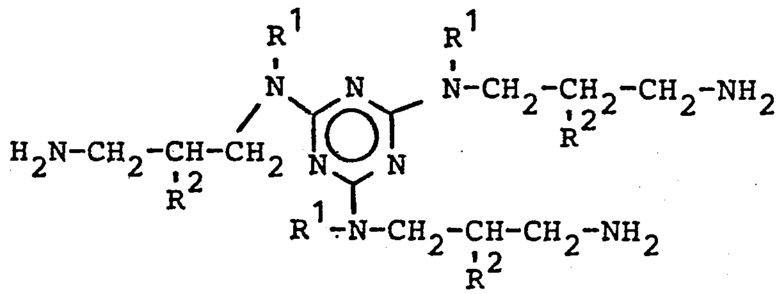

in welcher

$R^1$ für einen Alkyl-, Cycloalkyl- oder Arylrest steht und

$R^2$ für Wasserstoff oder einen Alkylrest steht.

2. Polyamine der in Anspruch 1 genannten Formel, für welche

$R^1$ für einen Alkylrest mit 1 bis 6 Kohlenstoffatomen, einen Cyclohexylrest oder einen Phenylrest steht und

$R^2$ für Wasserstoff oder eine Methylgruppe steht.

3. Polyamine der in Anspruch 1 genannten Formel, für welche

$R^1$ für einen Alkylrest mit 1 bis 4 Kohlenstoffatomen steht und

$R^2$ für Wasserstoff oder eine Methylgruppe steht.

4. Verfahren zur Herstellung von Polyaminen gemäß Anspruch 1, dadurch gekennzeichnet, daß man Trinitrile der Formel

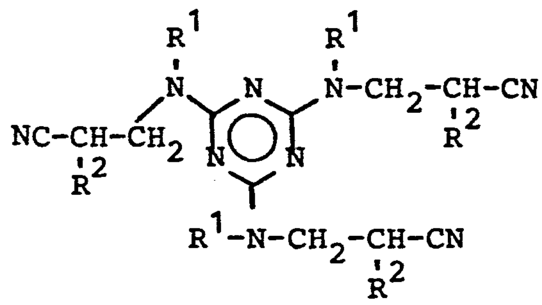

in welcher

$R^1$ und $R^2$ die in Anspruch 1 genannte Bedeutung haben,

in an sich bekannter Weise katalytisch unter Überführung der Nitrilgruppen in Aminogruppen hydriert.

0151927

Europäisches Patentamt

# EUROPÄISCHER RECHERCHENBERICHT

Nummer der Anmeldung

EP 85100117.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 553 137 (BASF)<br>* Formel I *<br>-- | 1 | C 07 D 251/70 |
| A | DE - A1 - 2 653 834 (ARMJANSKIJ)<br>* Seite 4 (Formel) *<br>-- | 1 | |
| A | CHEMICAL ABSTRACTS, Vol. 88, No. 17, 24. April 1978, Columbus, Ohio, USA<br>RUTTY, C.J.; CONNERS, T.A. In vitro studies with hexamethylmelamine. page 17, column 1, Zusammenfassung-Nr. 115 093s<br>& Biochem.Pharmacol. 1977, 26(24), 2385-91<br>-- | 1 | **RECHERCHIERTE SACHGEBIETE (Int. Cl.4)** |
| A | CHEMICAL ABSTRACTS, Vol. 87, No. 19, 7. November 1977, Columbus, Ohio, USA<br>CUMBER, A.J.; ROSS, W.C.J. Analogs of hexamethylmelamine. The anti-neoplastic activity of derivatives with enhanced water solubility. page 14, column 1, Zusammenfassung-Nr. 145 542j<br>& Chem.-Biol. Interact. 1977, 17(3), 349-57<br>---- | 1 | C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 17-04-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82